# EUROPÄISCHE PATENTANMELDUNG

(11) **EP 2 095 807 A1**
(43) Veröffentlichungstag der Anmeldung: **02.09.2009**
(21) Anmeldenummer: 08102116.4
(22) Anmeldetag: 28.02.2008
(51) Int. Cl.: A61K 8/31, A61K 8/37, A61K 8/42, A61Q 19/02, A61Q 19/06

(54) **Creme gegen Cellulite und Sommersprossen**

(71) Anmelder: Nguyen-Petersen, Chanh-Dinh, 50678 Köln (DE)
(72) Erfinder: Nguyen-Petersen, Chanh-Dinh, 50678 Köln (DE)
(74) Vertreter: Helbing, Jörg

(57) **Zusammenfassung**

Gegenstand der vorliegenden Erfindung ist eine harnstoffhaltige Hautcreme zur Behandlung von Cellulite und zum Entfernen von Sommersprossen.

## Beschreibung

Gegenstand der vorliegenden Erfindung ist eine harnstoffhaltige Hautcreme zur Behandlung von Cellulite und zum Entfernen von Sommersprossen.

Im Bereich der kosmetischen Hautcremes ist bekannt, dass Zusätze des in der Haut vorkommenden Harnstoffes in Hautcremes zu einer besonderen Eignung der Hautcreme zur Verwendung auf trockener Haut führen. Dies liegt darin begründet, dass Harnstoff eine hohe Wasserspeicherfähigkeit aufweist. Bei einer Anwendung harnstoffhaltiger Hautcremes auf trockener Haut sorgt der Harnstoffgehalt der Hautcreme daher für eine Zufuhr fehlender Feuchtigkeit. Da die feuchtigkeitsbindende Wirkung von Harnstoff in Hautcremes seit längerem bekannt ist, existieren auf dem Markt bereits eine Vielzahl harnstoffhaltiger Cremes, die als Kosmetika anzusehen sind. Beispielhaft sei hierfür das unter der Bezeichnung "Eucerin^{®} 5 % Urea Gesichtscreme" vertriebene Produkt der Beiersdorf AG, Hamburg, genannt, welches neben den Inhaltsstoffen Wasser, Glyzerin und Triisostearin einen Anteil von 5 Gew.-% an Harnstoff enthält. Als Verwendungshinweise gibt der Hersteller u.a. an: Zur täglichen Pflege bei trockener, spannender rauer und schuppiger Gesichtshaut, auch zur therapiebegleitenden Pflege z. B. bei Neurodermitis und Psoriasis.

Die kosmetische Wirksamkeit der Creme wird dahingehend erklärt, dass bei Anwendung der genannten kosmetischen Hautcreme das Feuchtigkeitsbindevermögen der Haut schnell und langanhaltend erhöht wird.

Wie sich aus den oben genannten Verwendungshinweisen bereits ergibt, ist ebenfalls bekannt, dass harnstoffhaltige Salben, d.h. Emulsionen mit geringerem Wassergehalt, sehr gut zur Behandlung der unangenehmen Nebenwirkungen von Neurodermitis und Psoriasis eignen. Hierbei ist in erster Linie die Linderung von Beschwerden zu nennen, die im Zusammenhang mit trockener Haut auftreten, so z. B. Juckreiz, übermäßige Schuppenbildung, Rissbildung, etc.

Die Verwendung von Harnstoffen sowohl in Kosmetikcremes als auch in medizinischen Cremes und Salben beruht stets auf der Erkenntnis, dass Harnstoff als Wirkstoff in einer Creme oder Salbe den Feuchtigkeitsgehalt trockener haut zu erhöhen vermag. Darüber hinausgehende Erkenntnisse über weitere Wirkungen von Harnstoff, die insbesondere eine gezielte Verwendung harnstoffhaltiger Cremes oder Salben nahe legen würden, gibt es bislang nicht.

Allen bislang im kosmetischen Bereich als Feuchtigkeitscremes angebotenen harnstoffhaltigen Hautcremes ist gemeinsam, dass ihr Harnstoffgewichtsanteil in der Gesamtzusammensetzung im allgemeinen bis zu 5 % beträgt.

Hingegen weisen die Hautcremes bzw. -salben aus dem medizinischen Bereich, die Harnstoff im Wirkstoffanteil aufweisen, einen deutlich erhöhten Harnstoffgehalt auf, der bis zu 12 % betragen kann. Diese harnstoffhaltigen, medizinischen Hautcremes weisen im allgemeinen einen gegenüber dem Wasseranteil deutlich erhöhten Fettanteil auf, wodurch sich eine salbenartige Konsistenz ergibt. Eine Verwendung derartiger Cremes und Salben ist praktisch nur noch im medizinischen Bereich möglich, da stark fetthaltige Salben deutliche kosmetische Nachteile wie das Hervorrufen fettglänzender Haut aufweisen.

In der DE A 102 41541 wurde eine harnstoffhaltige Hautcreme mit einem Wirkstoffanteil an Harnstoff von 8 bis 25 Gew.-% beschrieben, die zur Behandlung von Pigmentstörungen und altersbedingter Falten geeignet ist.

Ein weiteres Hautproblem für das bisher noch keine erfolgreiche Behandlung gefunden wurde, ist Cellulite (Dermopanniculosis deformans). Die Wirkstoffe/Wirkstoffkombinationen, die hier eingesetzt werden, reichen von aktivem Koffein über Mäusedorn bis hin zu Apfelbaumextrakt. Allen diesen Mitteln ist gemeinsam, dass ihre Wirksamkeit von der Stiftung Warentest , S. 21, 4/2003, als mangelhaft eingestuft wurde.

Es wurde nunmehr gefunden, dass eine wie in der DE-A-10242541 beschriebenen Hautcreme ebenfalls sehr gute Effekte bei der Behandlung von Cellulite und zum Entfernen von Sommersprossen aufweist.

Bei regelmäßiger Anwendung über einen verhältnismäßig kurzen Zeitraum führte die Anwendung der harnstoffhaltigen Creme zu einer deutlich sichtbaren Verringerung der cellulitischen Haut und einer Entfärbung der Sommersprossen. Die Erfindung betrifft somit
(1) die Verwendung einer Hautcreme, die wenigstens 8 Gew.-% Harnstoff enthält, zur Behandlung von Cellulite oder zur Entfernung von Sommersprossen der menschlichen Haut;
(2) ein Verfahren zur Behandlung von Cellulite oder zur Entfernung von Sommersprossen der menschlichen Haut umfassend die topische Applikation einer harnstoffhaltigen Hautcreme wie vorstehend unter (1) definiert auf den zu behandelnden Hautbereich; und
(3) eine harnstoffhaltige Hautcreme wie vorstehend unter (1) definiert zur Behandlung von Cellulite oder zur Entfernung von Sommersprossen der menschlichen Haut.

Es stellt sich daher bei einer regelmäßigen Anwendung einer Hautcreme mit einem Gewichtsanteil von Harnstoff von 8 bis 25 Gew.-% innerhalb einer verhältnismäßig kurzen Zeitspanne, die sich im Bereich einiger Wochen bewegt, ein kosmetischer Erfolg in Form einer Hautglättung und verringerter Sommersprossen dar. Die behandelte Haut wirkt verjüngt und erfrischt. So bildet sich Cellulite innerhalb von 4 Wochen merklich zurück. Bei einer entsprechend langen Behandlung von Sommersprossen waren diese merklich aufgehellt. Die besten Erfolge zeigten sich bei Salben mit Harnstoffkonzentration von größer oder gleich 20 Gew.-%.

Im Rahmen der vorliegenden Anmeldung wird unter dem Begriff "Hautcreme" sowohl Cremes (Wasser-in-Öl-Emulsion mit relativ niedrigem Wassergehalt) als auch Salben (Öl-in-Wasser-Emulsion), Lotionen (z. B. Wasser-in-Öl-Emulsion mit relativ hohem Wassergehalt) sowie Gele (ölfreie Zubereitungen) verstanden werden.

Eine Rezeptur für eine kosmetisch verwendbare Hautcreme mit entsprechendem Harnstoffanteil besteht aus einer Cremegrundlage und einem Wirkstoffanteil. Hierbei weist die Cremegrundlage vorzugsweise die folgenden Inhaltsstoffe auf:
1. gereinigtes Wasser,
2. weiße Vaseline,
3. Glycerolmonostearate,
4. Propylenglycol,
5. mittelkettige Triglyceride,
6. Cetylalkohol,
wobei der Anteil des gereinigten Wassers mehr als 35 Gew.-% der Cremegrundlage beträgt.

Der Wirkstoffanteil enthält den Harnstoff, wobei der Harnstoffanteil in der Creme vorzugsweise mindestens 8 Gew.-% und maximal 25 Gew.-% beträgt.

Als bevorzugte Untergrenze für eine schnelle Wirksamkeit der erfindungsgemäßen Hautcreme hat sich ein Mindestanteil von 10 Gew.-% Harnstoff in der Hautcreme als günstig erwiesen.

Als besonders geeigneter Harnstoffanteil hat sich daher ein Harnstoff von 8 bis 30, vorzugsweise von 10 bis 25 Gew.-% in der Hautcreme herausgestellt. Besonders bevorzugt wird dabei ein Harnstoffanteil zwischen 20 und 25 Gew.-% verwendet.

Um kosmetisch unerwünschte Effekte bei der Anwendung der erfindungsgemäßen Hautcreme wie z. B. übermäßig glänzende Haut zu vermeiden, hat es sich herausgestellt, dass der Anteil der weißen Vaseline in der Cremegrundlage vorteilhaft unter 28 Gew.-% beträgt.

Darüber hinaus hat sich herausgestellt, dass die als Emulgatoren verwendeten Glycerolmonostearate vorteilhaft Anteile von Glycerolmonostearate 60 sowie Makrogell-20 Glycerolmonostearate umfassen.

Um eine möglichst nebenwirkungsfreie Anwendbarkeit der erfindungsgemäßen Hautcreme auch auf empfindlicher Haut beispielsweise von allergiegefährdeten Personen zu ermöglichen, sollte die erfindungsgemäße Hautcreme keine Konservierungsstoffe enthalten. Im Rahmen der durchgeführten Untersuchungen konnte festgestellt werden, dass eine Hautcreme mit der erfindungsgemäßen Rezeptur auch ohne Zusatz zusätzlicher Konservierungsstoffe weitgehend unabhängig von den Zubereitungsbedingungen der Hautcreme über einen Zeitraum von einem Jahr verwendbar war. Werden erhöhte Anforderungen an die Sterilität bei der Zubereitung der erfindungsgemäßen Hautcreme erfüllt, so kann die Haltbarkeit der Hautcreme auch ohne Konservierungsstoffe ohne weiteres bis auf über zwei Jahre hinaus ausgedehnt werden.

Aus den vorgenannten Gründen einer möglichst guten Verträglichkeit der erfindungsgemäßen Hautcreme sollte diese ebenfalls keine PH-puffernden Substanzen wie Milchsäure oder Natriumlactat sowie keine Entschäumer wie Dimeticon enthalten. Es hat sich bei der praktischen Erprobung der erfindungsgemäßen Hautcreme erwiesen, dass zur Herstellung der erfindungsgemäßen Hautcreme auch in größeren Mengen, beispielsweise im Rahmen einer automatisierten großtechnischen Produktion, keine PH-puffernden Substanzen und keine Entschäumer erforderlich sind.

Die Hautcreme hinterlässt nach dem Auftragen keine glänzende Fettschicht auf der Haut und lässt sich hervorragend mit warmem Wasser auswaschen.

Die genannten Inhaltsstoffe sind kostengünstig auch im größeren Umfang verfügbar, kostenintensive Zusatzstoffe sind nicht enthalten.

Die Herstellung der beschriebenen Hautcreme ist sowohl in Form einer Einzelzubereitung als auch im großtechnischen Maßstab möglich.

Die erfindungsgemäße Hautcreme weist damit neben ihren erfindungsgemäßen kosmetischen Vorteilen den weiteren Vorteil einer besonders hohen Verträglichkeit auf, so dass sie ohne weiteres auch von allergiegefährdeten Personen oder Personen mit akuten Hauterkrankungen verwendet werden kann.

Weitere Vorteile und Merkmale der erfindungsgemäßen Hautcreme sowie der erfindungsgemäßen Verwendung einer harnstoffhaltigen Hautcreme ergeben sich aus den nachfolgenden Beispielen, die nicht einschränkend zu verstehen sind.

### Beispiel 1: Herstellung der Hautcreme

Aus den folgenden Bestandteilen wird eine Cremegrundlage hergestellt:
1. 40 Gew.-% gereinigtes Wasser,
2. 25,5 Gew.-% weiße Vaseline,
3. 10 Gew.-% Propylenglykol,
4. 7,5 Gew.-% mittelkettige Triglyceride,
5. 7 Gew.-% Makrogel-20-Glycerolmonostearat,
6. 6 Gew.-% Cethylalkohol,
7. 4 Gew.-% Glycerolmonostearat 60

Glycerolmonostearat 60, Cetylalkohol, mittelkettige Triglyceride und weißes Vaselin werden im Wasserbad auf etwa 60°C erhitzt und anteilsweise mit der auf die gleiche Temperatur erwärmten Mischung von Macrogol-20-Glycerolmomostearat, Propylenglykol und gereinigtem Wasser versetzt. Die Creme wird bis zum Erkalten ständig gerührt und das verdunstete Wasser ergänzt.

Nach der Herstellung der Cremegrundlage wird in diese in einigen Tropfen Wasser gelöster Harnstoff bis zu einem Gewichtsanteil von 20 Gew.-% eingearbeitet, wodurch sich der Wasseranteil geringfügig erhöht und eine stabile Creme erhalten wurde.

### Beispiel 2: Behandlung von Cellulite

Zur Behandlung von Cellulite wurde die Hautcreme von Beispiel 1 bei vier Probanden zweimal täglich dünn auf die zu behandelnde Hautpartie an Bauch und Oberschenkel aufgetragen und leicht einmassiert. Ein Auswaschen der Creme ist nicht erforderlich.

Nach vier Wochen Behandlungszeit stellte sich bei allen Probanden eine deutliche Glättung der behandelten Hautpartien ein.

### Beispiel 3: Behandlung von Sommersprossen

Zur Behandlung von Sommersprossen im Gesicht wurde die Creme von Beispiel 1 zweimal täglich dünn auf die Haut aufgetragen und leicht einmassiert. Dabei sollte die zu behandelnde Hautpartie sauber und trocken sowie frei von anderen Cremes sein. Danach wird die Creme über eine Einwirkzeit von fünf bis zehn Minuten auf der Haut belassen. Schließlich wird die Creme vorteilhaft mit reinem warmen Wasser ausgewaschen.

Nach einer vierwöchigen Anwendung zeigte sich ein deutliches Verblassen der Sommersprossen.

## Patentansprüche

1. Verwendung einer Hautcreme, die wenigstens 8 Gew.-% Harnstoff enthält, zur Behandlung von Cellulite oder zur Entfernung von Sommersprossen der menschlichen Haut.

2. Verwendung nach Anspruch 1, wobei die Hautcreme bis zu 30, vorzugsweise bis zu 25 Gew.-%, Harnstoff enthält.

3. Verwendung nach Anspruch 1 oder 2, wobei die Hautcreme wenigstens 10 Gew.-% Harnstoff enthält, vorzugsweise 20 bis 25 Gew.-%, Harnstoff enthält.

4. Verwendung nach einem oder mehren der Ansprüche 1 bis 3, wobei die Hautcreme aus einer Cremegrundlage und einem Wirkstoffanteil besteht, wobei vorzugsweise
a) die Cremegrundlage die folgenden Inhaltsstoffe aufweist:
i) gereinigtes Wasser
ii) weiße Vaseline
iii) Glycerol-Monostearat
iv) Propylenglykol
v) mittelkettige Triglyceride
vi) Cetylalkohol,
wobei der Anteil des gereinigten Wassers in der Cremegrundlage mehr als 30 Gew.-% beträgt, und
b) der Wirkstoffanteil Harnstoff enthält, so dass der Harnstoffanteil in der Hautcreme wenigstens 8 Gew.-% beträgt.

5. Verwendung nach Anspruch 4, wobei der Anteil der weißen Vaseline in der Cremegrundlage weniger als 28 Gew.-% beträgt.

6. Verwendung nach Anspruch 4 oder 5, wobei der Glycerolmonostearat-Anteil in der Cremegrundlage Glycerolmonostearat 60 und Macrogel-20-Glycerolmonostearat umfasst.

7. Verwendung nach einem der Ansprüche 4 bis 6, wobei die Creme keine Konservierungsstoffe und/oder keine pH-Puffer enthält.

8. Verwendung nach einem der Ansprüche 4 bis 7, wobei die Creme keine Entschäumer enthält.

9. Verfahren zur Behandlung von Cellulite oder zur Entfernung von Sommersprossen der menschlichen Haut umfassend die topische Applikation einer harnstoffhaltigen Hautcreme wie in einem der Ansprüche 1 bis 8 definiert auf den zu behandelnden Hautbereich.

10. Harnstoffhaltige Hautcreme wie in einem der Ansprüche 1 bis 8 definiert zur Behandlung von Cellulite oder zur Entfernung von Sommersprossen der menschlichen Haut.
